(19) Europäisches Patentamt

European Patent Office

Office européen des brevets

(11) EP 1 063 522 A2

# (12) EUROPEAN PATENT APPLICATION

(43) Date of publication:
27.12.2000 Bulletin 2000/52

(51) Int. Cl.⁷: **G01N 29/18**, G01N 29/20, G01N 33/12

(21) Application number: 00304349.4

(22) Date of filing: 23.05.2000

(84) Designated Contracting States:
**AT BE CH CY DE DK ES FI FR GB GR IE IT LI LU MC NL PT SE**
Designated Extension States:
**AL LT LV MK RO SI**

(30) Priority: 22.06.1999 US 338459

(71) Applicant:
**Guigné International Ltd**
**Paradise, Newfoundland A1L 1C1 (CA)**

(72) Inventors:
• **Klein, Kenneth**
**Mount Pearl, Newfoundland A1N 3A7 (CA)**

• **Guigné, Jacques Y.**
**Paradise, Newfoundland A1L 1C1 (CA)**
• **Quanshun, Liu**
**Paradise, Newfoundland A1L 1C1 (CA)**
• **Cawthorne, Richard**
**Cornwall PE1 C0A, 1HC (CA)**

(74) Representative:
**Hackney, Nigel John et al**
**Mewburn Ellis,**
**York House,**
**23 Kingsway**
**London WC2B 6HP (GB)**

## (54) Ultrasonic seafood probe

(57) Apparatus for evaluating a part of a seafood animal such as a lobster claw, to determine the ratio of meat to water and other characteristics, including first and second transducers (12, 14) that are coupled to spaced locations on the part P of the animal so when the first transducer transmits sonic energy it passes through the animal part to the second transducer. A circuit (34) coupled to the second transducer analyzes detected sonic energy. In one system, the circuit calculates the velocity of sound through the part of the seafood animal. A higher velocity indicates a higher meat content. Another circuit includes an indicator that indicates the amplitude of sound detected by the second transducer, especially the amplitude of sound of the predominant frequency generated by the first transducer, that has passed directly through the animal part. A lower amplitude indicates a higher meat content. A pressure sensor (124) automatically activates the first transducer to generate sonic energy, when the transducers are pressed with a predetermined force against the part of the seafood animal. The transducers can be mounted on a glove (92) between the thumb (T) and an opposed finger (F), so the part can be tested when it is picked up by a person wearing the glove.

FIG. 1

EP 1 063 522 A2

Printed by Xerox (UK) Business Services
2.16.7 (HRS)/3.6

**Description**

Seafood animals such as lobsters, bivalves and oysters, vary in the quantity and quality of their meat. For example, a lobster with a high percentage of meat is likely to be healthy and survive a long trip to a distant buyer, and provides a better tasting product. A lobster with lesser meat percentage may be of value only for consumption within a short period of time, and even then will not taste as good. After lobsters are harvested, the lobster processor is faced with the task of determining the meat content and quality of the incoming lobsters on an individual basis. Although evaluations can be based on the color, weight, and other characteristics noted without probing the inside of the lobster, it would be desirable if a more accurate and/or objective evaluation of the quantity and quality of a seafood animal part, could be obtained.

In accordance with one embodiment of the present invention, apparatus is provided for making an accurate and/or objective evaluation of the quality and quantity of meat of a seafood animal, in a rapid and moderate cost manner. The apparatus includes first and second transducers and a means for coupling the transducers to opposite sides of a part of a seafood animal. The first transducer is activated to pass acoustic energy through the part of the animal to the second transducer, which generates a signal representing the acoustic energy it detects. The signal from the second transducer is delivered to a circuit that generates a signal representing the quality (e.g. ratio of mass of solids to mass of solids plus liquids) of meat of the part of the seafood animal.

One apparatus includes a distance sensor which generates a signal representing the distance between locations on the animal part through which acoustic energy is passed, the signal being delivered to the circuit that evaluates the quality of the meat. The circuit includes a velocity-calculating circuit part which generates a signal representing the velocity of the acoustic energy through the part of the seafood animal. The circuit for evaluating the quality of meat can include an indicator that indicates the amplitude of signals from the second transducer, and that indicates a high quality when the amplitude is below a predetermined first level, and that indicates a low quality when the amplitude is above a predetermined second level.

One apparatus for pressing the transducers towards the opposite sides of the part of the seafood animal to be evaluated, includes a glove with a thumb sleeve for receiving the thumb of a worker's hand and a finger sleeve for receiving a finger of the worker which lies opposite his thumb. The transducers are mounted on the sleeves, so a worker can pick up the seafood animal and press the transducers against the opposite sides of a part of an animal with his thumb and opposed finger. A pressure sensor senses when a predetermined pressure against the animal part is exceeded, to automatically energize the first transducer to generate acoustic energy.

The novel features of the invention are set forth with particularity in the appended claims. The invention will be best understood from the following description when read in conjunction with the accompanying drawings.

BRIEF DESCRIPTION OF THE DRAWINGS

Fig. 1 is a front elevation view and partial block diagram of apparatus for evaluating the condition of a part of a seafood animal, constructed in accordance with one embodiment of the present invention, the seafood animal part representing a lobster crusher claw of high quality.

Fig. 2 is a sectional view representation a lobster crusher claw of low quality.

Fig. 3 is a view of a portion of the apparatus of Fig. 1, showing the apparatus being calibrated.

Fig. 4 is a sectional view of the lobster claw of Fig. 1 and including three graphs, including a graph showing the amplitude vs. time characteristics of acoustic energy delivered to one side of the lobster claw, the amplitude vs. time characteristics of the acoustic energy detected at an opposite second side of the lobster claw, and the amplitude vs. frequency characteristics of the acoustic energy picked up at the second side of the lobster claw.

Fig. 5 is a side elevation view of a glove of a second embodiment of the invention, for rapidly passing acoustic energy through a lobster claw.

Fig. 6 is a block diagram showing circuitry for generating signals representing the quality of lobster claw meat by measuring the velocity of acoustic energy through the lobster claw and by measuring the amplitude of acoustic energy detected by the second transducer.

DESCRIPTION OF THE PREFERRED EMBODIMENTS

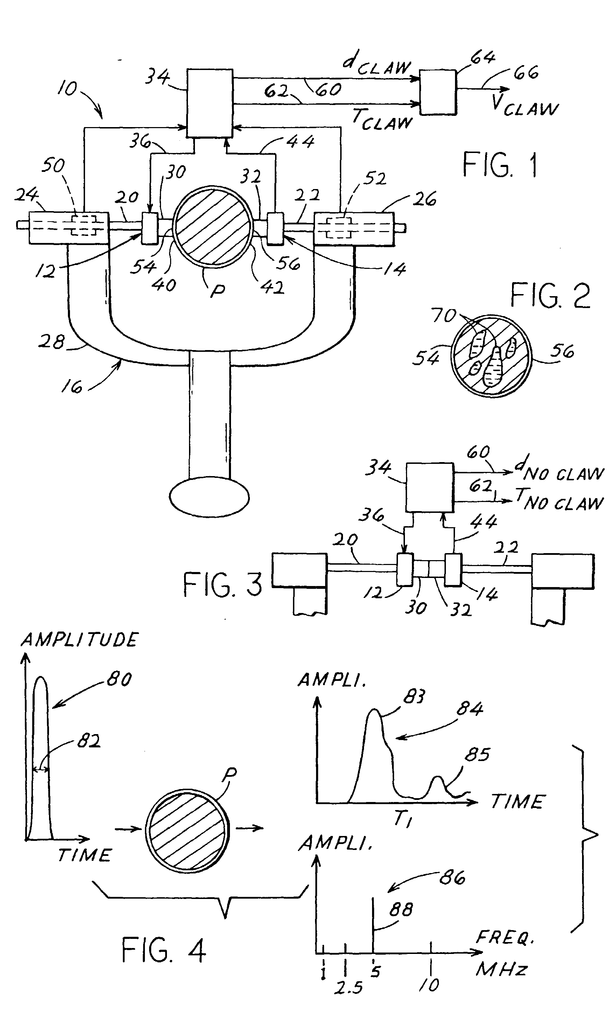

Fig. 1 illustrates apparatus 10 for evaluating the condition of a part P of a seafood animal such as a lobster crusher claw. The apparatus includes first and second acoustic transducers 12, 14 and a holder 16 that supports the transducers. The holder includes a pair of rods 20, 22 that slide within ends 24, 26 of a holder base 28 to enable the transducers to be moved toward and away from the part. A pair of acoustic couplings or horns 30, 32 couple each transducer to an opposite side of the animal part P and sometimes can be considered part of the transducer. A first circuit 34 generates an electrical signal (e.g. a pulse) on output line 36, which is connected to the first transducer 12 to cause the first transducer to generate acoustic energy. The acoustic energy passes through the horn 30 to the first side 40 of a seafood animal part P to the second side 42, and through the second horn 32 to the second transducer 14. The second transducer 14 generates an electrical signal on line 44, which is delivered to the first circuit 34. The first circuit 34 can then process the signal received by the second transducer 14 to generate signals indicating the quality of meat in the seafood animal part P.

In Fig. 1, a pair of position sensors 50, 52 lie in the ends of the holder base, to sense the separation of the horns 30, 32, to thereby detect the separation between the locations 54, 56 where acoustic energy is applied and detected. The first circuit 34 generates a signal on line 60 which is identified as $d_{claw}$ which represents the distance that the acoustic energy passed when passing through the claw of the lobster part P. The first circuit also delivers a signal on line 62 which indicates the time period required for sound to pass through the part P, between the locations 54, 56. The output on line 62 is designated as $T_{claw}$. A second circuit 64 receives the two signals on lines 60, 62 respectively representing the distance that the sound traveled within the seafood animal part P and the time of transit of the acoustic energy through the part, and generates a signal on output line 66 which represents the velocity of sound through the seafood animal part. This signal is designated $V_{claw}$, in that it represents the velocity of sound through the claw.

Fig. 3 illustrates the apparatus of Fig. 1, during a calibration process. The rods 20, 22 are moved towards each other until the horns 30, 32 abut one another. Then the first circuit 34 transmits an energizing signal through line 36 to the first transducer 12 to energize it, and detects the output on line 44 from the second transducer 14. The outputs on lines 60 and 62 represent the distance $d_{no\ claw}$ and $T_{no\ claw}$ representing the distance between the transducers when there is no claw present and the time required for acoustic energy to pass between the transducers when no claw is present. The actual velocity of acoustic energy through the claw in one example is given by the following equation:

$$V_{claw} = \frac{(d_{claw} - d_{no\ claw})}{\Delta T_{claw} - \Delta T_{no\ claw}} = \frac{10cm - 5cm}{79.4_{usec} - 50_{usec}}$$

$$= \frac{5 \times 10^{-2} m}{29.4 \times 10^{-6\ sec}} = 17 \times 10^2\ m/sec = 1,700\ m/sec$$

Where $V_{claw}$ equals the velocity of sound through the claw and the other variables are as given above.

For a very healthy claw, $V_{claw}$ has been found to be about 1,750 meters per second, while for a low quality claw the velocity would be somewhat closer to the velocity of sound through water of 1,450 meters per second. The claw of Fig. 2 might produce a velocity of 1,700 m/sec. which indicates high quality. A lower velocity indicates less dense material in the claw, which can indicate more pockets of water 70 in the claw and which also may indicate an unhealthy lobster. The circuit 34 can be used to detect reflections of the acoustic energy (e.g. from a pocket 70 to the lobster claw wall at location 54 and from there to the second location 56), to further evaluate the part.

Fig. 4 illustrates a method for evaluating the quality of meat for a lobster crusher claw P, where the output of the second transducer is evaluated by detecting its frequency components. In Fig. 4, the graph 80 represents the characteristics of a pulse of current that drives the first transducer (12 in Fig. 1). The pulse may be of short duration 82 such as 1/5th microsecond, which results in ultrasonic acoustic energy being transmitted through the animal part P by a transducer 12 with fast reaction time. The predominant frequency transmitted is at 5 MHZ. Graph 84 represents the output of the second transducer (14 in Fig. 1) showing the variation in amplitude with time. Graph part 83 represents the direct travel of sound through the animal part to the second transducer, while part 85 represents a reflection. Graph 86 represents the Fourier transform of the graph 84 during a time TI which begins when the pulse 80 is transmitted and that ends before most internal reflections in the animal part are received by the second transducer. The graph 86 shows that the acoustic energy received by the second transducer at part 83, has a moderate amplitude at the predominant frequency of 5MHz and a much smaller amplitude at other frequencies. The amplitude of sound transmitted through the animal part and detected by the second transducer indicates the quality of meat in the seafood animal part P.

Information about both velocity $V_{claw}$ and the amplitude at the predominant frequency (e.g. 5MHz) can provide a good assessment of the quality of the seafood part. A higher velocity indicates a higher quantity of meat, because of the higher velocity of sound through most solids, including meat, than through water. The detection of a larger amplitude of acoustic energy at the prime frequency, for a given amplitude of the transmitted pulse 80, indicates a lower quantity of meat. This is because meat absorbs a higher percent of sound than does water. Experiments conducted by applicant affirm that a higher quantity (and quality) of meat is indicated by a higher velocity of sound, and by the detection of a lower percentage of the sound that was applied to the side of the animal part lying opposite the location where sound is detected.

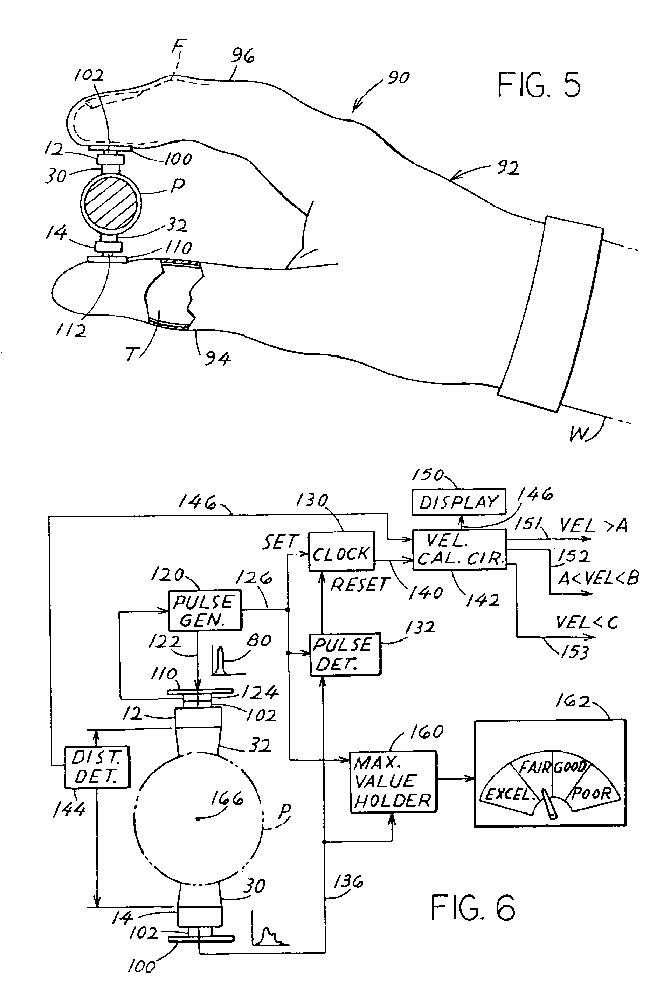

Fig. 5 shows an apparatus 90 that facilitates coupling the transducers 12, 14 to the animal part P. The apparatus includes a glove 92 with a thumb sleeve 94 for receiving the thumb T of the wearer, and a finger sleeve for receiving an opposed finger F of the wearer, such as the index or middle finger of the wearer. The finger sleeve 96 holds a mount 100 on which the first transducer 12 is mounted, through a dampener 102, and with a horn 30 mounted on the transducer. The thumb sleeve 94 is similarly constructed, with a mount 110, dampener 112, and horn 32. The wearer W can grasp the part P of the seafood animal and immediately cause sonic energy to pass between the transducers 12, 14 by way of the part, to produce the evaluations described above. Preferably a pressure sensor senses when the wearer has pressed the animal part P between his thumb and opposed finger with a predetermined force such as at least 5 pounds, to then automatically activate the circuit that energizes the first transducer 12 and to set the circuits that evaluate the output from the second transducer 14. The worker may be holding the seafood animal with his other hand when he grasps the part with the glove. The sleeves do not have to cover the entire thumb or other finger, but only hold themselves in place. A distance measuring device such as a cylinder and piston extending between the two sleeves beside the lobster claw, measures distance.

Fig. 6 is a block diagram showing circuitry for operating the transducers of Fig. 5. A pulse generator 120 generates a pulse such as indicated at 80 on line 122, which energizes the transducer 12 to generate acoustic energy that is passed through the seafood animal part P. While the worker could operate a pedal or other switch, applicant prefers to provide a pressure sensor 124 which senses the force with which the transducers are pressed towards the animal part P. When the pressure increases past a predetermined level such as 5 pounds, the pressure sensor 124 automatically activates the pulse generator 120 to generate the pulse 80. A portion of the pulse or a signal representing the beginning of the pulse, is transmitted over line 126 to a clock 130 to set it, to a pulse detector 132, and to a circuit 160 that detects the maximum level of signals from the second transducer.

During a period such as two hundred microseconds when the second transducer 14 detects the ultrasonic energy, the output of the transducer 14 is delivered through line 136 to the pulse detector 132, which detects the leading edge of the pulse and which sends a signal to the clock to reset it. By subtracting the time between the setting and resetting of the clock, a signal over line 140 representing the time required for the transit of the pulse is delivered to a velocity calculating circuit 142. The circuit 142 will have earlier received a signal representing the sound transit time with the horns touching one another, as in Fig. 3, and takes the difference to calculate the time for sonic energy to pass only through the animal part P. A distance detector 144 detects the distance between the transducers 12, 14. Such distance detector can include the two position sensors 50, 52 in Fig. 1. The output of the distance detector on line 146 is delivered to the velocity calculating circuit 142. The distance during calibration as in Fig. 3 when the horns touch, has previously been delivered to the circuit 142, so the circuit can calculate the distance through the animal part P as the difference in the detected distance when no animal part was present and when the animal part is present. The velocity calculating circuit 142 delivers a signal on line 146 to a display 150 which displays the velocity of sound through the animal part.

The circuit 142 also has three outputs 151, 152, and 153 which indicate whether the quality and quantity of meat is acceptable. If the velocity is greater than a predetermined level A such as 1,690 meters per second, thee the signal 151 is generated, which may sound a high pitched sound, indicating to the person holding the animal part that the meat is probably of an acceptable level. If the velocity is less than a predetermined level C such as 1,600 meters per second, then the signal on line 53 may generate a low pitched sound indicating that the part is of unacceptable quality. If the velocity is between the levels A and B such as 1,600 to 1,690 meters per second, then a signal on line 152 may energize a buzzer of moderate pitch to indicate this to the person making the evaluation. Of course, the signals can be green, yellow, and red lights.

A piezoelectric transducer used at 14 is sensitive to a limited band of frequencies. The particular transducer 14 that is used is most sensitive to the primary frequency (5MHz) of the pulse 80 applied to the first side of the animal part, and therefore acts as a filter to pass only that frequency. The circuit 160 holds the maximum amplitude of the signal detected by detecting transducer 14. The circuit 160 is reset by the pulse on line 126 and holds the highest output from transducer 14 received during a predetermined time (e.g. 300 microseconds) after the pulse. The output from circuit 160 (representing maximum amplitude) is continually delivered to a display 162. In one example, the pulse generator 120 delivers a pulse of predetermined maximum amplitude (e.g. 100 volts and 0.05 ampere) to the first transducer 12. A certain maximum amplitude output is produced by the detecting transducer 14. Test objects, one representing an animal part with a high meat content and the other representing an animal part with low meat content, are tested to determine the instantaneous maximum resulting outputs on line 136 (e.g. 10 millivolts for the part with high meat content and 14 millivolts for the part with low meat content). The tests performed using the two test objects can be used to calibrate the indicator 162. For example, with the "good" and "bad" test objects resulting in maximum outputs on line 136 of 10mv and 14mv, respectively, the indicator 162 can be programmed to move a needle on a dial between extremes of "Excellent" (10mv) and "Poor" (14 mv), and to indicate grades ("Good" and "Fair") in between the extremes.

It is also possible to generate a Fourier transform of the input and output signals to compare the amplitudes of different frequencies and the times of detections (to detect reflections).

While it is simplest to apply the transducers (or horns connected to the transducers) to diametrically opposite sides of the part, the transducers can be applied to any two locations, which are preferably spaced at least 90° about the axis 166 of the part so that the transducers are coupled to locations at opposite sides of the part. It is possible to use two or more detecting transducers, to detect sonic energy that has passed along different paths through the animal part. It is possible to transmit continuous sonic energy, such as a sine wave of numerous cycles, instead of pulses. In general, applicant prefers to pass ultrasonic acoustic energy (above 20KHz) through the animal part. Of course the outputs from the velocity-indicating circuit, the analyzing circuit, and any other circuit, can be combined to provide an output that indicates the quality of the animal part.

Applicant prefers to apply ultrasonic energy of a frequency less than 10MHz. Attenuation generally increases with frequency, so at high frequencies (e.g. near and above 10MHz) the output of the receiving transducers are very low and partially masked by noised. Also, at frequencies of about 10 MHz and higher, the output of the detecting transducer is affected by the curvature of the lobster claw. Applicant prefers to use frequencies of at least about 1 MHz so that directly transmitted sound can be readily distinguished from sound that was internally reflected.

Applicant has conducted tests on 54 lobsters, and found that measurements of sound velocity and relative amplitude of the detecting transducer output at the primary frequency were good indicators of lobster meat quality. Applicant performed the tests with transmitting transducers driven at primary frequencies of 5MHz and 2MHz. Applicant found it best to apply the transducers to the crusher claw of the lobster. The tail is of more complicated construction than the claws, resulting in more difficulty in analyzing and less consistent results. Applicant found that either the pincer claw or crusher claw of the lobster could be tested, but found greater reproducibility when the crusher claw was used and prefers the crusher claw.

In the lobster industry, the quality of a lobster is often given by the term "Meat Content" or "MC", where:

$$MC = \frac{\text{weight of meat}}{\text{weight of meat} + \text{weight of fluids}}$$

where the lobster is weighed and then freeze dried to get rid of fluids, and weighed again. The difference before and after freeze drying is the "weight of fluid". Then, the meat is scooped out to obtain "weight of meat." The equation is not affected by the weight of the shell.

While applicant generally prefers to use acoustic transducers that are energized by electrical signals, it should be noted that there are acoustic transducers available which are mechanically activated, without requiring an electrical signal. The second or detecting transducer is always one which generates an electrical signal for analysis. Better acoustic coupling of a transducer horn to a side of a lobster is often obtained by applying a gel between them.

Thus, applicant provides an apparatus for evaluating the condition of a part of a seafood animal, by energizing a first transducer to produce acoustic energy that is transmitted through the part, and by detecting acoustic energy at an opposite side of the part and analyzing the detected acoustic energy by a circuit which generates a signal representing the quality or relative quantity of meat of the seafood animal part. The circuit means for analyzing the second transducer, can include a velocity-calculating circuit that is coupled to the first and second transducers that respectively transmit and receive acoustic energy, and to a distance sensor which senses the distance along which the acoustic energy passed in passing through the seafood animal part. The circuit generates a signal representing the velocity of the acoustic energy. Generally, a higher acoustic velocity indicates better quality meat while a lower velocity indicates lower quality meat. A second circuit generates signals representing the variation in amplitude of detected sound (at the dominant frequency transmitted to the animal part) represented by the output of the second transducer, relative to the amplitude of transmitted sound generated by the first transducer (or the energy applied to the first transducer). A glove device can be used which has sleeves including a thumb sleeve for receiving the thumb of the wearer and a finger sleeve for receiving the opposed finger of the wearer. The transducers can be mounted one on each sleeve, so a worker can grasp the animal part between the thumb and opposed finger to automatically press the transducers toward opposite sides of the animal part. A pressure sensor can be used to automatically initiate a pulse or other acoustic energy so the wearer can initiate an evaluation by merely pressing against opposite sides of the animal part with a predetermined moderately high force.

Although particular embodiments of the invention have been described and illustrated herein, it is recognized that modifications and variations may readily occur to those skilled in the art, and consequently, it is intended that the claims be interpreted to cover such modifications and equivalents.

## Claims

1.  Apparatus for evaluating the condition of a part (P) of a seafood animal, comprising:

a first transducer (12) for generating acoustic energy, a second transducer (14) for detecting acoustic energy and for generating electrical signals representing the detected acoustic energy, and means (16, 92) for coupling said transducers to spaced locations (54, 56) on said part of the seafood animal to transmit acoustic energy between said transducers by way of said part of the seafood animal;

circuit means (64, 142, 160) coupled to at least said second transducer, for generating a signal representing the quantity of meat of said part of the seafood animal.

2. The apparatus described in claim 1 including:

a distance sensor (50, 52, 144) which is coupled to said transducers and which generates a signal representing the distance between said spaced locations;
said circuit means includes a velocity-calculating circuit (64, 142) that is coupled to said transducers and to said distance sensor, and which generates a signal representing the velocity of said acoustic energy passed through said part of the seafood animal between said spaced locations.

3. The apparatus described in claim 2 wherein:

said velocity calculating circuit is constructed to generate a first signal (151) indicating a favorable condition of said part of the sea food animal when the velocity of said acoustic energy is greater than a first predetermined velocity, and to generate a second signal (153) indicating an unfavorable condition of said part of the seafood animal when the velocity is less than a second predetermined velocity.

4. The apparatus described in claim 1 wherein:

said circuit means includes a display (162) indicating the amplitude of sound energy detected by said second transducer.

5. The apparatus described in claim 4 wherein:

said first transducer device is constructed to generate a sonic pulse having a primary component of a predetermined prominent frequency (88) which has a greater amplitude than components of any other frequency;
said display is constructed to indicate a favorable condition of the part of the seafood animal when the amplitude of said prominent frequency of acoustic energy from said second transducer is less than a first predetermined level, and to indicate an unfavorable condition of the part of the sea food animal when the amplitude of said prominent frequency of acoustic energy from said second transducer is greater than a second predetermined level.

6. The apparatus described in claim 1 wherein:

said first transducer is electrically energizable; and including first and second mounts (100, 110) that respectively support said first and second transducers;
a pressure sensor (124) lying in series with one of said transducers, said pressure sensor constructed to generate a signal representing the force with which the corresponding transducer is being pushed toward the corresponding location on said part of the seafood animal;
an electrical circuit (120) coupled to said pressure sensor, said circuit being activatable by a predetermined force sensed by said pressure sensor, to produce electrical signals that energize said first transducer to produce acoustic energy.

7. The apparatus described in claim 1 including:

a glove (92) having finger sleeves (94, 96) for receiving the thumb (T) and an opposed finger (F) of the wearer, with each of said transducers mounted on a different one of said sleeves, so the wearer presses said transducers against said spaced locations when the wearer grasps said part of the seafood animal.

8. Apparatus for evaluating the condition of a part (P) of a seafood animal, comprising:

a first transducer (12) which is activatable to generate acoustic energy, a second transducer (14) for detecting acoustic energy and generating electric signals representing the detected acoustic energy, and means (16, 92) for coupling said transducers to spaced locations (54, 56) on said part of the seafood animal to transmit acoustic energy between said transducers by way of said part of the seafood animal;

a circuit (64, 142, 160) that is coupled to said second transducer and that generates signals indicating the condition of said part of the seafood animal according to the signals generated by said second transducer;

said means for coupling including a pressure sensor (124) which activates said first transducer upon the detection of a force of at least a predetermined value on at least one of said transducers as it presses against a location on said part of the seafood animal.

9. Apparatus for evaluating the condition of a part (P) of a seafood animal, comprising:

a glove (92) that is designed to lie around the hand of a worker, with said glove having thumb and finger sleeve means (94, 96) for respectively receiving the thumb (T) and another finger (F) of the worker to grasp said part of a seafood animal;

first and second acoustic transducers (12, 14) mounted respectively on said thumb and finger sleeve means and positioned to be coupled to first and second locations on said part of the seafood animal when the worker grasps the part between the worker's thumb and other finger that are received in said thumb and finger sleeve means, to enable the passage of acoustic energy from said first transducer through said part to said second transducer;

an electrical circuit (34, 64, 120, 142, 160) which is connected to said first transducer to energize it to generate acoustic energy, said circuit being connected to said second transducer and constructed to analyze signals detected by said second transducer and generate signals indicating the quality of said part of the seafood animal.

10. Apparatus for evaluating the condition of a part (P) of a seafood animal, comprising:

a first transducer (12) for generating acoustic energy, a second transducer (14) for detecting acoustic energy and generating electric signals representing the detected acoustic energy, and means (30, 32) for coupling said transducers to spaced locations (54, 56) on said part of the seafood animal to transmit acoustic energy between said transducers by way of the said part of the seafood animal;

a velocity calculating circuit (64, 142) that is coupled to said transducers and which generates a signal representing the velocity of said acoustic energy through said part of a seafood animal that lies between said spaced locations.

11. Apparatus for evaluating the condition of a part (P) of a seafood animal, comprising:

a first transducer device (12) for generating acoustic energy, a second transducer (14) for detecting acoustic energy and generating electric signals corresponding to the detected acoustic energy, and means (30, 32) for coupling said first transducer device and said second transducer to spaced locations (54, 56) on said part of the seafood animal to transmit acoustic energy from said first transducer device to said second transducer by way of said part of the seafood animal;

an indicator (162) coupled to said second transducer and which is constructed to indicate high quality when the amplitude of the output of said second transducer is at a lower level and to indicate a lower quality than said high quality when the output of said second transducer is at a level that is higher than said low level, when any other measured quantities are constant.

12. A method for evaluating the quality of meat of a part of a seafood animal, comprising:

acoustically coupling first and second transducers to first and second locations on opposite sides of said part of the seafood animal;

energizing said first transducer to generate acoustic energy which is coupled to said first location and detecting electrical signals produced by said second transducer which represent acoustic energy at said second location which has passed through said part of a seafood animal between said locations;

analyzing said electrical signals produced by said second transducer and using said analysis to generate a signal representing the quality of meat of said part of the seafood animal.

13. The method described in claim 12 including:

measuring the distance between said locations; and wherein

said step of analyzing includes calculating the time for the acoustic energy to pass between said locations, by using the measurement of distance between said locations and the time for the acoustic energy to pass, to generate a signal representing the velocity of acoustic energy through said part;

using said signal representing velocity to generate said signal representing the quality of meat by indicating a high quality when the velocity is at a high level and by indicating a lower quality that said high quality when the velocity is at a low level that is less than said high level.

14. The method described in claim 12 wherein:

said seafood animal is a lobster, and said step of coupling includes pressing horns on said transducers against opposite sides of the crusher claw of the lobster.

15. The method described in claim 12 wherein:

said first transducer is electrically energizable; and including
sensing the compressive force on at least one of said transducers, and energizing said first transducer upon said compressive force reaching a predetermined level.

16. The method described in claim 12 wherein:

said step of coupling said first and second transducers includes mounting said transducers one on a thumb sleeve and the other on an opposed finger sleeve of a glove, placing the sleeves on the thumb and opposed finger of a person, and grasping said part of the seafood animal between said transducers while they are mounted on said sleeves.

FIG. 1

FIG. 2

FIG. 3

FIG. 4

FIG. 5

FIG. 6